# EUROPEAN PATENT SPECIFICATION

(11) **EP 4 453 064 B1**
(45) Date of publication and mention of the grant of the patent: **03.12.2025**
(21) Application number: 22830898.7
(22) Date of filing: 22.12.2022
(51) Int. Cl.: C08G 63/688, C08F 220/38, C08G 59/30

(54) **SULFUR-BRIDGED THERMOPLASTIC POLYESTERS AND THERMOSETS FROM FURFURAL**
SCHWEFELVERBRÜCKTE THERMOPLASTISCHE POLYESTER UND DUROPLASTE AUS FURFURAL
POLYESTERS THERMOPLASTIQUES À PONT SOUFRÉ ET PLASTIQUES THERMODURCIS ISSUS DU FURFURAL

(30) Priority: 22.12.2021 FI 20216329
(43) Date of publication of application: 30.10.2024
(73) Proprietor: Oulun Yliopisto, 90014 Oulun Yliopisto (FI)
(72) Inventor: HEISKANEN, Juha, 90014 Oulun yliopisto (FI); KAINULAINEN, Tuomo, 90014 Oulun yliopisto (FI); AHMED, Asmaa, 90014 Oulun yliopisto (FI)
(74) Representative: Laine IP Oy
(86) International application number: PCT/FI2022/050867
(87) International publication number: WO 2023/118667

(56) References cited:
- WO-A1-2020/254715
- KAINULAINEN TUOMO P. ET AL: "Application of Furan-Based Dicarboxylic Acids in Bio-Derived Dimethacrylate Resins", ACS APPLIED POLYMER MATERIALS, vol. 2, no. 8, 30 June 2020 (2020-06-30), pages 3215 - 3225, XP093025372, ISSN: 2637-6105, Retrieved from the Internet <URL:http://pubs.acs.org/doi/pdf/10.1021/acsapm.0c00367> DOI: 10.1021/acsapm.0c00367

## Description

### FIELD

The present disclosure relates generally to polymer chemistry and particularly to the syntheses of novel sulfur-bridged difuran polyesters. In certain aspects, the present disclosure also relates to food and beverage packaging materials and curable resins.

### BACKGROUND

Recently, there has been an increased focus on obtaining polymeric materials derived from renewable resources. This growing trend is aiming at finding replacements to fossil-based resources and materials such as poly(ethylene terephthalate), PET, a high performance plastic that is especially widely used in packaging due to its gas barrier properties, transparency, and mechanical strength.

There has been growing interest towards the utilization of renewable lignocellulose-based feedstocks in the production of biobased chemicals and materials. One of the most attractive biochemicals is furfural, a dehydration product of hemicellulose-based C5 sugars. Furfural contains an aldehyde group and the aromatic five-atom furan ring. The single ring substituent of furfural has restricted its utilization in the production of many polymers since two polymerizable groups are often required (e.g., two carboxylic acid functionalities). 2,2'-Bifuran-5,5'-dicarboxylic acid (BFDCA) is one state-of-the-art, furfural-derived dicarboxylic acid which can be used as a monomer in the production of e.g., polyesters with properties such as high gas barrier and intrinsic UV-screening (Kainulainen et al., 2018 and 2020 and Ahmed et al., 2021, WO2020/254715). To prepare BFDCA, two furfural-derived molecules are connected using a palladium catalyzed coupling reaction, which forms a new C-C bond between the two furan rings. Effective methods that accomplish this are direct coupling (Kainulainen et al., 2018) and reductive homocoupling (Lei et al., 2020). It is also known to use furan-based dicarboxylic acid in bio-derived dimethacrylate resins (Kainulainen et al.,, ACS Appl. Polym. Mater., 2020, 2, 3215-3225).

In this invention, furfural-derived molecules are connected with a sulfur bridge to create a series of novel bifunctional monomers. Notably, the sulfur-bridged monomers are synthesized without expensive transition metal catalysts and the method allows straightforward utilization of furfural as a raw material.

### SUMMARY

The present invention provides novel furfural-derived bifunctional compounds. The sulfur-containing furan polyesters of the invention are excellent barrier materials and provide some degree of screening in the UV-A region. The novel monomers can be utilized for example in the production of sustainable bioplastics, both thermoplastic polyesters and curable thermosets.

Accordingly, the present invention provides a sulfur-bridged difuran polyester comprising a dicarboxylate component and a diol component, wherein the dicarboxylate component and/or the diol component comprise(s) a sulfur-bridged difuran structure.

In certain aspects, the present invention provides a package or packaging material comprising said polymer.

In certain aspects, the present invention provides a film, yarn fiber or coating comprising or consisting of said polymer.

In other related aspects, the present invention provides a method for preparing a sulfur-bridged difuran, the method comprising the steps of:
a) reacting halogenated furfurals according to Formula 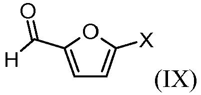
   wherein X is independently Cl, Br or I;
   with a sulfur donor in order to produce a sulfur-bridged difuran 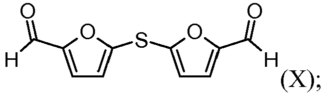
b) selectively oxygenating the sulfur-bridged difuran obtained from step a);
c) subjecting the oxygenated sulfur-bridged difuran to esterification reaction in order to produce one of the following compounds: 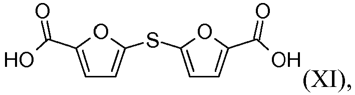 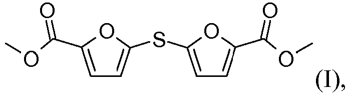 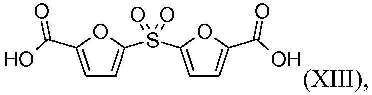 and

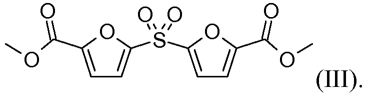

In other related aspects, the present invention provides a method for preparing a sulfur-bridged difuran, the method comprising the steps of:
a) reacting halogenated furfurals according to Formula IX 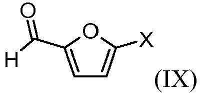
   wherein X is independently Cl, Br or I;
   with a sulfur donor in order to produce a sulfur-bridged difuran having the Formula X 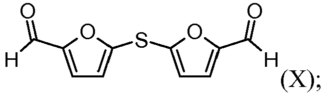
b) selectively oxygenating the aldehyde groups of the sulfur-bridged difuran obtained from step a);
c) esterifying the sulfur-bridged difuran diacid obtained from step b);
d) subjecting the esterified sulfur-bridged difuran thus obtained to oxygenation reaction in order to produce one of the following compounds: 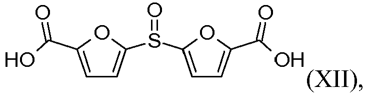 and

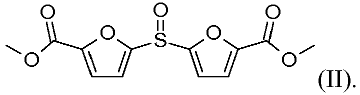

In other aspects, the present invention provides a sulfur-bridged difuran compound comprising or consisting of a structure of any of the following formulas: 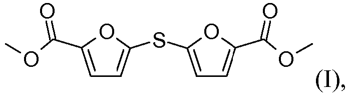 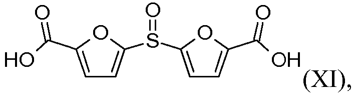 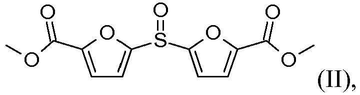 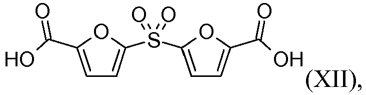 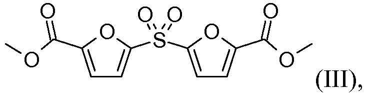 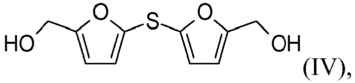 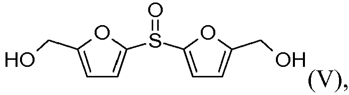 and

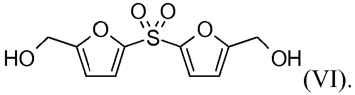

In certain aspects, the present invention is directed to the use of the sulfur-bridged difuran compound in preparation of polyester with improved oxygen barrier and/or ultraviolet light (UV) blocking properties compared to poly(ethylene terephthalate) based polymers.

In certain aspects, the present invention provides a curable resin composition comprising at least one of the following sulfur-bridged difuran compounds: 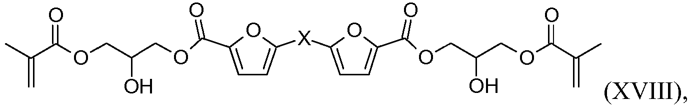 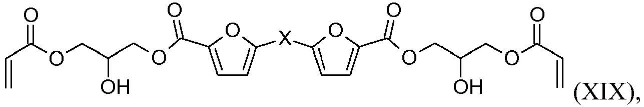 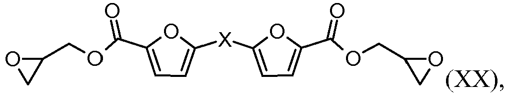 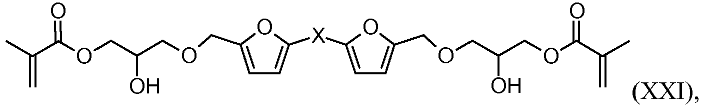 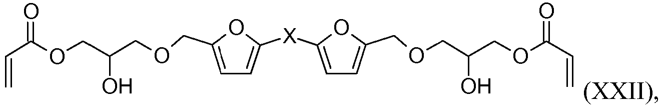 and 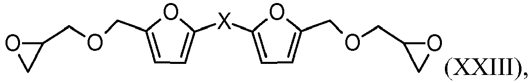 wherein X is selected from the group consisting of -S-, -SO-, and -SO₂-.

In certain aspects, the present invention is directed to a method for preparing a sulfur-bridged difuran diol comprising the step of reacting the sulfur-bridged difuran aldehyde or ester according to Formula 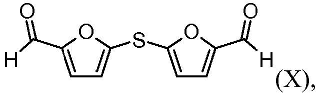 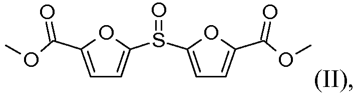 or 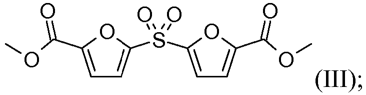 with sodium borohydride (NaBH₄) or lithium aluminum hydride (LiAlH₄) in order to produce one of the following compounds:

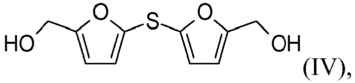

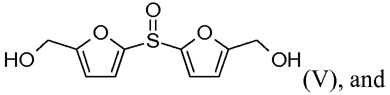

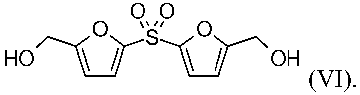

More specifically, the invention is mainly characterized by what is stated in the independent claims.

### BRIEF DESCRIPTION OF THE DRAWING

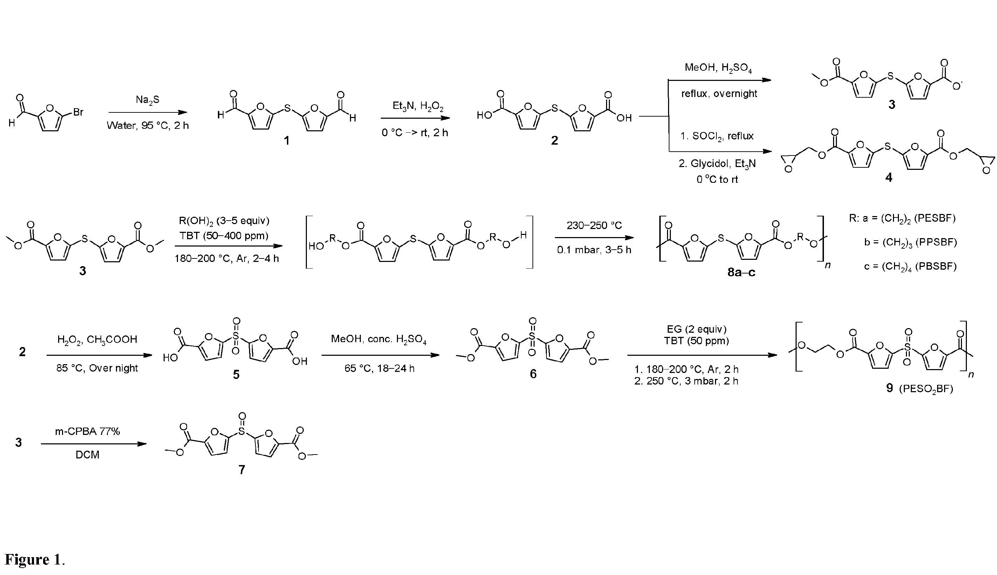

**Figure 1****.** General synthesis route of the monomer and polyesters of the present disclosure.

### DETAILED DESCRIPTION OF EMBODIMENTS

As used herein, the term "polymer" describes an organic substance composed of a plurality of repeating structural units (backbone units) covalently connected to one another. The term "polymer" as used herein encompasses organic and inorganic polymers and further encompasses one or more of a homopolymer, a copolymer or a mixture thereof (a blend). The term "homopolymer" as used herein describes a polymer that is made up of one type of monomeric units and hence is composed of homogenic backbone units. The term "copolymer" as used herein describes a polymer that is made up of more than one type of monomeric units and hence is composed of heterogenic backbone units. The heterogenic backbone units can differ from one another by the pendant groups thereof.

The term "polyester" as used herein is inclusive of polymers prepared from multiple monomers that are referred to herein as copolyesters. Terms such as "polymer" and "polyester" are used herein in a broad sense to refer to materials characterized by repeating moieties or units. The polyesters as described herein may have desirable physical and thermal properties and can be used to partially or wholly replace polyesters derived from fossil resources, such as poly(ethylene terephthalate), PET. The term "repeating unit," as used herein with reference to polyesters refers to an organic structure having a dicarboxylic/dicarboxylate component residue and a diol component residue bonded through an ester linkage.

The term "residue" as used herein, means the organic structure of the monomer in its as-polymerized form as incorporated into a polymer, e.g., through an esterification, transesterification, and/or poly condensation reaction from the corresponding monomer(s). Throughout the specification and claims, reference to the monomer(s) in the polymer is understood to mean the corresponding as-polymerized form or residue of the respective monomer. For purposes herein, it is to be understood that by reference to a copolyester comprising a dicarboxylic/dicarboxylate component and a diol component, the dicarboxylic/dicarboxylate and diol components are present in the polymer in the as-polymerized (as-condensed) form, where it is understood the alkyl ester groups in the starting material are not present in the polyester, except sometimes in end groups of the polyester.

Unless otherwise clear from context, percentages referred to herein are expressed as percent by weight based on the total composition weight.

Unless otherwise stated, properties that have been experimentally measured or determined herein have been measured or determined at room temperature. Unless otherwise indicated, room temperature is 25°C.

Unless otherwise stated, properties that have been experimentally measured or determined herein have been measured or determined at atmospheric pressure.

As used herein, the term "glass transition temperature (*T*_{g})" refers to a temperature range where a polymer changes from a hard, rigid or "glassy" state to a more pliable, compliant or "rubbery" state.

It must also be noted that, as used in the specification and the appended claims, the singular forms "a," "an" and "the" include plural referents unless otherwise specified.

The present invention is directed to a sulfur-bridged difuran polyester comprising a dicarboxylate component and a diol component, wherein the dicarboxylate component and/or the diol component comprise(s) a sulfur-bridged difuran structure, wherein the sulfur atom is attached to the two furan rings of the difuran with covalent sulfur-carbon bonds. Preferably, the said dicarboxylate component is any of the following formulas: 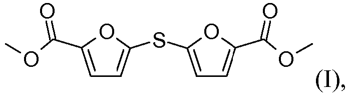 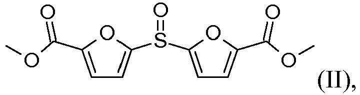 and

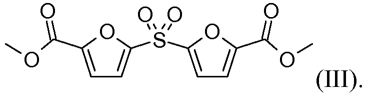

Preferably, the said diol component is any of the following formulas: 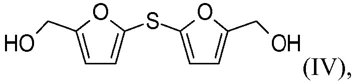 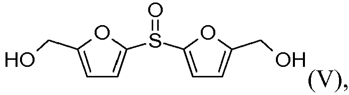 and

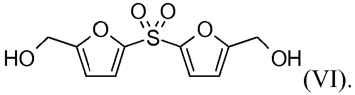

In some embodiment, the said polyester is a homopolyester comprising repeating units of one of the structures according to formula 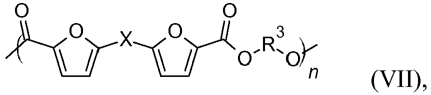
wherein X is independently selected from the group consisting of: -S-, -SO-, and -SO₂-;
wherein R³ is independently selected from the group consisting of: -(CH₂)₂-, -(CH₂)₃-, - (CH₂)₄-, -(CH₂)₅-, -(CH₂)₆-, -(CH₂)₇-, -(CH₂)₈-, 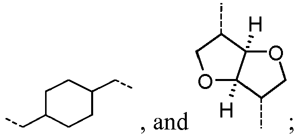 and
n is integer 1 or more, preferably between 1-200 or 5-200.

In some embodiment, the said polyester is a copolyester and said diol component is an aliphatic, cycloaliphatic or aromatic C₁-C₈ diol. Preferably, the said aliphatic, cycloaliphatic, or aromatic C₁-C₈ diol is selected from the group consisting of: ethylene glycol, 1,3-propanediol, 1,4-butanediol,1,4-cyclohexanedimethanol, and isosorbide.

In some embodiments, said polyester is a copolyester further comprising an aliphatic or cycloaliphatic C₃-C₈ dicarboxylic residue, an aromatic C₆-C₁₂ dicarboxylic residue or an aromatic C₆-C₈ dicarboxylic monomer residue as a repeating unit. Preferably, said aromatic C₆-C₈ dicarboxylic monomer residue is dimethyl terephthalate (DMT).

In some embodiments, the said copolyester comprises repeating units of at least one of the structures according to formula 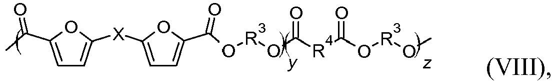
wherein X is selected from the group consisting of -S-, -SO-, and -SO₂-;
wherein each R³ is independently selected from the group consisting of -(CH₂)₂-, - (CH₂)₃-, -(CH₂)₄-, -(CH₂)₅-, -(CH₂)₆-, -(CH₂)₇-, -(CH₂)₈-, 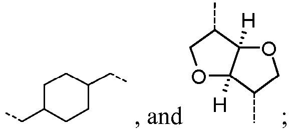 and
wherein R⁴ is selected from the group consisting of -(CH₂)₂-, -(CH₂)₃-, -(CH₂)₄-, - (CH₂)₅-, -(CH₂)₆-, and the following cyclic ring structures 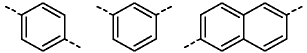 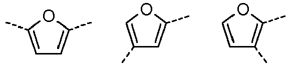 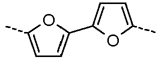 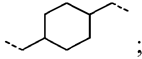 and
y and z are independently integer 1 or more, preferably 1-30. Preferably, the ratio of y:z is between 200:1 and 1:200. More preferably, said ratio is 90:10, 75:25, 50:50, 25:75, 10:90, 1:100 or any range between the listed ratios.

Having similar or better properties compared to PET (see Experimental Section below), a person skilled in the art would understand that the above described polyester can be applied to beverage bottles, food package films and other food package containers, cosmetics packages, detergent and washing agent containers, medicinal substance containers, and yarn fibers.

The present invention is thus also directed to a package or packaging material comprising a sulfur-bridged difuran polyester as defined above. Preferably, said package is a food package or a beverage container.

Further, the present invention is directed to a film, yarn fiber or coating comprising or consisting of the a sulfur-bridged difuran polyester as defined above.

The present invention is also directed to a method for preparing a sulfur-bridged difuran, the method comprising the steps of:
a) reacting halogenated furfurals according to formula 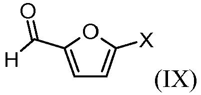
   wherein X is independently Cl, Br or I;
   with a sulfur donor in order to produce a sulfur-bridged difuran 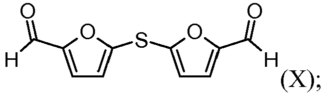
b) selectively oxygenating the sulfur-bridged difuran obtained from step a), preferably in the presence of H₂O₂/Et₃N and/or H₂O₂/CH₃COOH;
c) subjecting the oxygenated sulfur-bridged difuran to esterification reaction in order to produce one of the following compounds: 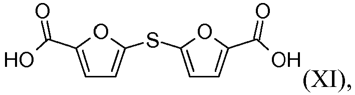 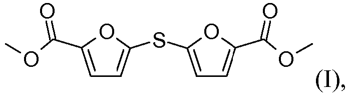 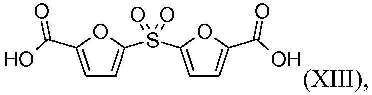 and

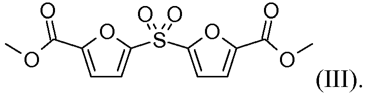

Preferably, the method comprises the steps of:
a) reacting halogenated furfurals according to Formula IX 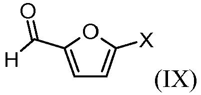
   wherein X is independently Cl, Br or I;
   with a sulfur donor in order to produce a sulfur-bridged difuran having the Formula X 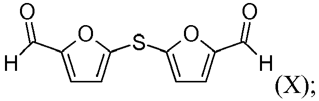
b) selectively oxygenating the aldehyde groups of the sulfur-bridged difuran obtained from step a), preferably in the presence of H₂O₂/Et₃N, in order to produce a sulfur-bridged difuran having the Formula XI; 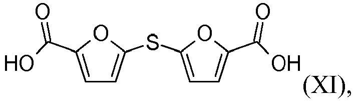
c) subjecting the oxygenated sulfur-bridged difuran thus obtained from step b) to esterification reaction in order to produce a sulfur-bridged difuran having the Formula I:

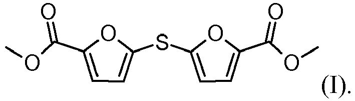

In another preferred embodiment, the method can comprise the steps of:
a) reacting halogenated furfurals according to Formula IX 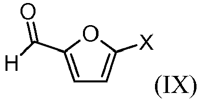
   wherein X is independently Cl, Br or I;
   with a sulfur donor in order to produce a sulfur-bridged difuran having the Formula X 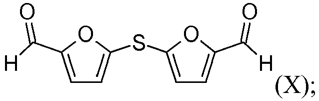
b) selectively oxygenating the aldehyde groups of the sulfur-bridged difuran obtained from step a), preferably in the presence of H₂O₂/Et₃N, in order to produce a sulfur-bridged difuran having the Formula XI; 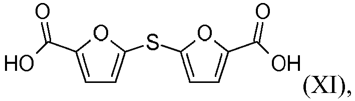
c) selectively oxygenating the sulfur atom of the sulfur-bridged difuran obtained from step b), preferably in the presence of H₂O₂/CH₃COOH, in order to produce a sulfur-bridged difuran having the Formula XIII; 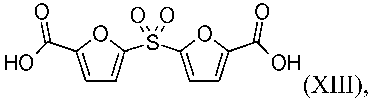
d) subjecting the oxygenated sulfur-bridged difuran thus obtained from step c) to esterification reaction in order to produce a sulfur-bridged difuran having the Formula III:

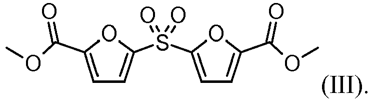

Alternatively, the method comprises the steps of:
a) reacting halogenated furfurals according to Formula IX 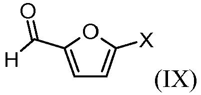
   wherein X is independently Cl, Br or I;
   with a sulfur donor in order to produce a sulfur-bridged difuran having the Formula X 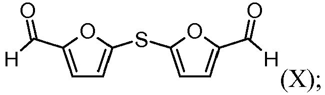
b) selectively oxygenating the aldehyde groups of the sulfur-bridged difuran obtained from step a), preferably in the presence of H₂O₂/Et₃N;
c) esterifying the sulfur-bridged difuran diacid obtained from step b);
d) subjecting the esterified sulfur-bridged difuran thus obtained to oxygenation reaction, preferably in the presence of m-CPBA, in order to produce one of the following compounds: 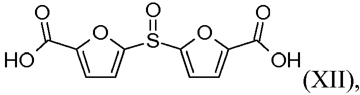 and

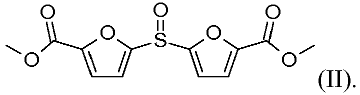

Thus, in a typical procedure sulfur-bridged difuran compounds are synthesized over three reaction steps as presented in Figure 1: I) synthesis of dialdehyde, II) oxidation of the dialdehyde to dicarboxylic acid, and III) esterification of the diacid to the dimethyl ester monomer.

In a preferred embodiment, said sulfur donor in step a) is sodium sulfide (Na₂S) or sodium thiosulfate (Na₂S₂O₃).

In some embodiments, the said method further comprises a step of performing a polycondensation process to the compounds produced in step c) in order to produce a polyester.

A typically useful procedure is a conventional two-step melt-polymerization method, such as generally also used in the production of PET. Thereby a mixture of the diol and dicarboxylic monomers are subjected to heating, in two stages. Thus, e.g., the mixture is first exposed to a temperature in the range of 140°C - 220°C, and thereafter to a temperature of 210°C - 260°C. Vacuum may be applied gradually, to obtain high molecular weight polyesters. Typically, the pressure applied during polycondensation step is subatmospheric, for example 0.01 to 900 mBar, for example about 0.1 to 100 mBar.

In preferred embodiments, the compound of 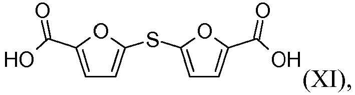 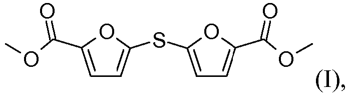 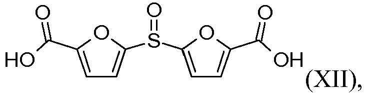 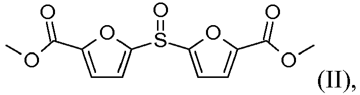 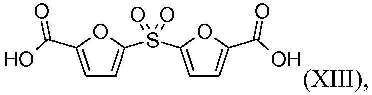 or

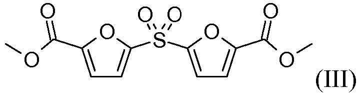

is reacted with any of the following compounds, or combinations thereof: 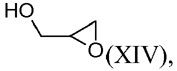 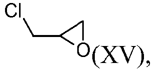 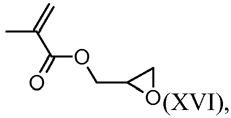 and

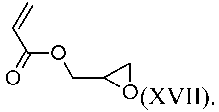

In another preferred embodiment, the compound of 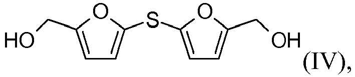 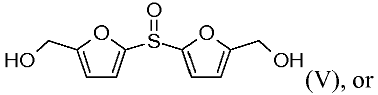 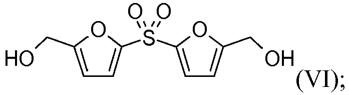 is reacted with any of the following compounds, or combinations thereof: 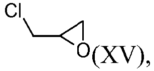 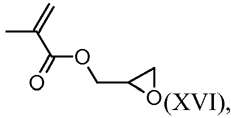 and

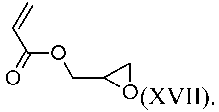

The invention is further directed to sulfur-bridged difuran compounds comprising or consisting of a structure of any of the following formulas: 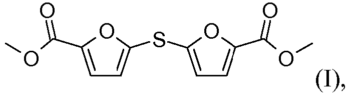 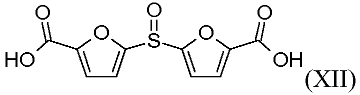 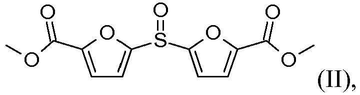 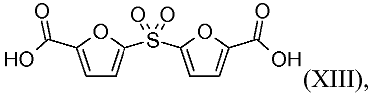 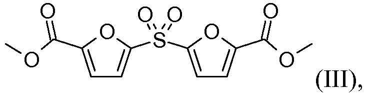 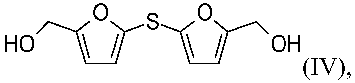 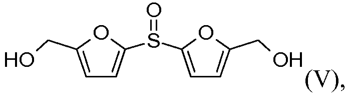 and

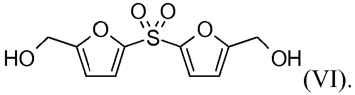

The present invention is also directed to use of said sulfur-bridged difuran compounds in preparation of polyester with improved oxygen barrier and/or ultraviolet light (UV) blocking properties compared to poly(ethylene terephthalate) based polymers.

The invention further provides a curable resin composition comprising at least one of the following sulfur-bridged difuran compounds: 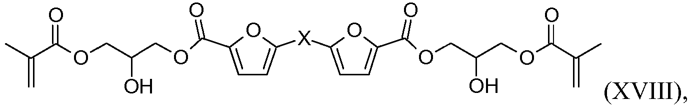 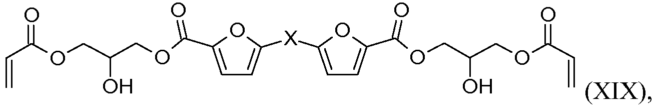 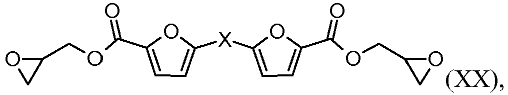 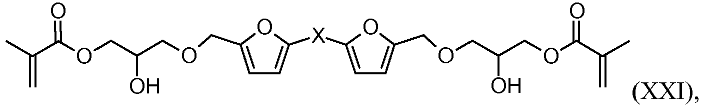 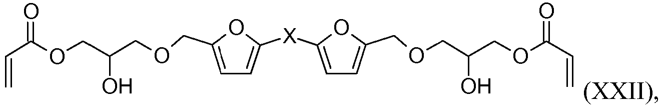 and 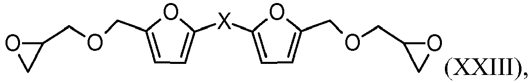 wherein X is selected from the group consisting of -S-, -SO-, and -SO₂-.

"Curable" refers to material that is obtained by irreversibly hardening a soft solid or viscous liquid prepolymer by cross-linking of individual chains of the polymeric substance of the resin under the influence of heat or suitable radiation, often under increased pressure. Preferably, the curable composition is cured by heating, or by UV radiation.

The invention is further directed to a composite, an adhesive, or a coating comprising at least one said curable sulfur-bridged difuran compounds.

Composite refers to composite material that comprises at least two components. These components may have notably dissimilar chemical or physical properties and are merged to create a material with properties unlike the individual elements. Within the finished structure, the individual elements remain separate and distinct, distinguishing composites from mixtures and solid solutions.

Adhesive refers to any composition that is capable of holding materials together by surface attachment that resists separation.

It will be obvious to a person skilled in the art that, as the technology advances, the inventive concept can be implemented in various ways. The invention and its embodiments are not limited to the examples described above but may vary within the scope of the claims.

### EXPERIMENTAL SECTION

### Materials and methods

Diols 1,2-ethanediol (99.8%), 1,3-propanediol (98%), and 1,4-butanediol (99%) were either purchased dry or distilled and stored under inert gas prior to use. Glycidol was distilled under reduced pressure prior to use. Otherwise, commercially available chemicals and solvents were used as received. Sodium sulfide hydrate (>60% Na₂S) was used as a source of sulfur in the reactions. Tetrabutyl titanate (TBT, 99%) was used as a catalyst for polycondensation reactions. Thin-layer chromatography was used for monitoring reactions using ethyl acetate and hexane (1:1 ratio) as an eluent. The compound numbering **1-9** used below refers to Figure 1 or Table 1.

*5,5'-sulfanediyldi(furan-2-carbaldehyde)* (**1**): 5-Bromofurfural (>98%, 10.71 g, 60 mmol) and sodium sulfide hydrate (0.55 equiv, 4.29 g) were reacted in deionized water (200 ml) at 95 °C for 2 hours. The cooled reaction mixture was filtered, and the obtained solid product was dried and purified by filtration through silica layer to afford yellow-orange powder (80%, 5.33 g). Melting point: 130 °C. ¹H NMR (400 MHz, CDCl₃, ppm): *δ* 9.62 (s, 2H), 7.25 (d, 2H, *J* = 3.5 Hz), 6.81 (d, 2H, *J =* 3.5 Hz). ¹³C NMR (100 MHz, CDCl₃, ppm): *δ* 177.3, 155, 147.8, 121.5, 118.7. HRMS m/z calculated for C₁₀H₆O₄NaS [M + Na]⁺: 244.9879, found 244.9877.

*5,5'-sulfanediyldi(furan-2-carboxylic acid)* **(2):** Compound **1** (12 g, 54 mmol) and triethylamine (40 mL) were mixed in a 250 mL two-necked flask, equipped with a magnetic stirring bar and a thermometer. The flask was placed in an ice bath, and once the reaction mixture temperature reached 0 °C, 30% hydrogen peroxide solution (4 equiv, 22 mL) was added dropwise while keeping the reaction temperature below 20 °C. After the addition of H₂O₂, the reaction flask was removed from the ice bath and the mixture was stirred at room temperature for 2 hours. Excess triethylamine was distilled off under reduced pressure. The reaction mixture was then diluted with 200 mL of deionized water and acidified (pH 1-2) with conc. hydrochloric acid. The formed off-white precipitate was filtered, washed with deionized water, rinsed with ethanol, and dried under vacuum to yield the product (95%, 13.03 g). ¹H NMR (400 MHz, DMSO-*d*₆, ppm): *δ* 13.44 (broad s, 2H), 7.27 (d, 2H, *J =* 3.4 Hz), 7.01 (d, 2H, *J =* 3.4 Hz). ¹³C NMR (100 MHz, (CD₃)₂SO, ppm): *δ* 159, 148.2, 145.1, 119.7, 119.6. HRMS *m*/*z* calculated for C₁₀H₇O₆S [M+ H]⁺: 254.9957, found 254.9958.

*Dimethyl 5,5'-sulfanediyldi(furan-2-carboxylate)* **(3):** Compound **2** (5.08 g, 20 mmol) was mixed with anhydrous methanol (150 mL) and 97% sulfuric acid (2 equiv, 2.23 mL). The mixture was refluxed overnight. The crude product was filtered, washed with cold methanol, and dried under suction to yield yellow crystals (5.4 g). The crude product was purified by sublimating at 170 °C under reduced pressure (<1 mbar), where the product was collected as white crystals in (97%, 5.21 g). Melting point: 153 °C. ¹H NMR (400 MHz, CDCl₃, ppm): *δ* 7.17 (d, 2H, *J* = 3.5 Hz), 6.74 (d, 2H, *J =* 3.5 Hz), 3.90 (s, 6H). ¹³C NMR (100 MHz, CDCl₃, ppm): *δ* 158.3, 147, 146, 119.3. 118.5, 52.2. HRMS m/z calculated for C₁₂H₁₁O₆S [M + H]⁺: 283.0270, found 283.0271.

*Diglycidyl ester of 5,5'-sulfanediyldi(furan-2-carboxylic acid)* (**4**): Compound **2** (2.06 g, 8 mmol) and SOCl₂ (3 equiv, 1.77 mL) were reacted in refluxing dichloromethane under argon. After overnight refluxing, the solution was filtered and evaporated to yield the crude diacyl chloride (2.29 g, 97%). The diacyl chloride, dissolved in dichloromethane (100 mL) cooled to 0 °C in an ice bath, was mixed with triethylamine (2.25 equiv, 2.77 mL). Under argon, a solution of glycidol in dichloromethane (2.25 equiv, 2.35 mL) was slowly introduced into the mixture over 20 min. Cooling was removed, and the reaction was allowed to continue at room temperature for 1 h. The reaction mixture was then extracted with deionized water, saturated NaHCO₃ solution, and brine. The organic layer was dried over anhydrous Na₂SO₄, filtered through a thin layer of silica gel, and evaporated to dryness to give the product (2.64 g, 92%). Melting point: 70 °C. ¹H NMR (400 MHz, CDCl₃, ppm): *δ* 7.20 (d, *J*=3.4 Hz, 1 H), 6.74 (d, J=3.4 Hz, 1 H), 4.62 (dd, *J*=12.2, 3.2 Hz, 1 H), 4.14 (dd, *J*=12.2*,* 6.4 Hz, 1 H), 3.31 (td, *J*=6.5, 3.1 Hz, 1 H), 2.89 (t, *J*=4.5 Hz, 1 H), 2.71 (dd, *J*=4.9, 2.7 Hz, 1 H).

*5,5'-Sulfonyldi(furan-2-carboxylic acid) (5):* Compound 2 (4.06 g, 16 mmol) was reacted with 30% H₂O₂ solution (30 mL, 25 equiv) in acetic acid (46 mL) at 85 °C overnight. The reaction mixture was cooled down and filtered, and the solid product dried under vacuum (3.40 g, 74%). ¹H NMR (400 MHz, DMSO-*d*₆, ppm): *δ* 14.2 (broad s, 2H), 7.43 (d, 2H, *J =* 3.5 Hz), 7.25 (d, 2H, *J =* 3.5 Hz). HRMS m/z calculated for C₁₀H₆O₈NaS [M + Na]⁺: 308.9675, found 308.9674.

*Dimethyl 5,5'-sulfonyldi(furan-2-carboxylate)* **(6):** Compound **5** (2.86 g, 10 mmol) was mixed with excess of methanol (150 mL) and 97% sulfuric acid (2 equiv, 1.15 mL) and refluxed overnight. The reaction system was allowed to cool down before the product was filtered. The product was recrystallized from methanol and subsequently purified by filtration through a silica layer to afford white crystals (2.93 g, 93%). ¹H NMR (400 MHz, CDCl₃, ppm): *δ* 7.43 (d, 2H, *J* = 3.4 Hz), 7.25 (d, 2H, *J* = 3.4 Hz), 3.92 (s, 6H). HRMS m/z calculated for C₁₂H₁₀O₈NaS [M+Na]⁺: 336.9988, found 336.9977.

*Dimethyl 5,5'-sulfinyldi(furan-2-carboxylate)* **(7):** Compound **3** (2.25 g, 8 mmol) was dissolved in CH₂Cl₂ (40 mL) in 100 mL round flask and cooled down to 0 °C. 77% m-CPBA (1.79 g, 8 mmol) in CH₂Cl₂ (20 mL) was added to the flask dropwise over 30 min. The reaction mixture was stirred for 5 h at -4 - 0 °C. Then, the reaction mixture was extracted twice with 50 mL portions of 10% NaHCO₃ solution. The organic phase was dried over MgSO₄, filtered, and evaporated to dryness. The mixture was purified through via column chromatography (silica) using hexane and ethyl acetate (1:3) as an eluent to afford the pure sulfoxide as white crystals (1.15 g, 43%). Melting point = 118 °C. ¹H NMR (400 MHz, CDCl₃, ppm): δ 7.27 (d, 2H, *J* = 3.4 Hz), 7.17 (d, 2H, *J =* 3.4 Hz), 3.92 (s, 6H). HRMS m/z calculated for C₁₂H₁₁O₇S [M + H]⁺: 299.0219, found 299.0220.

*Polyester synthesis* **(8a-c)**: Tetrabutyl titanate (50-400 ppm wt.% relative to monomer weight) was dissolved in dry toluene (0.5 mL) and mixed with the diol (3-5 equiv) along with **3** (2.25 g, 8 mmol) in a 100 mL round-bottom flask equipped with a magnetic stirring bar. The flask was connected to a distillation bridge and the reaction system was evacuated and filled with argon gas 5 times. The transesterification step was started at 180 °C. The temperature was raised gradually to 200 °C over 2-4 h until no more methanol was received. For the polycondensation step, the pressure was gradually reduced to 3 mbar over 1 h and the reaction temperature was increased to 230-250 °C. Once the excess of diol was distilled off and the pressure reduced to 0.1 mbar, the polymerization was run for 3-5 h. The polymeric product was dissolved in 1,1,1,3,3,3-hexafluoroisopropanol. The addition of methanol precipitated the polyester, which was filtered and washed with cold methanol. The product was dried under vacuum at 65 °C to yield a white-to-off white fibrous polymer. Polyester samples (ca. 10 mg) were dissolved in a mixture of TFA-*d* and CDCl₃ (1:3, v/v) for NMR measurements.

*Polyester synthesis* **(9):** Monomer **6** (2.51 g, 8 mmol) was added to a 100 mL reaction flask with TBT (50 ppm wt% equiv), dissolved in 0.5 mL of dry toluene, and mixed with ethylene glycol (2 equiv, 0.89 mL) and Irganox 1010 antioxidant (1000 ppm mol% relative to monomer ratio). The flask was connected to a distillation bridge and the reaction system was evacuated and filled with argon gas 3 times. The monomer was transesterified by ethylene glycol for 2 h at 180 °C and at 200 °C. The reaction pressure was decreased gently from 1000 mbar to 3 mbar during 1 h to avoid sublimation of short oligomers. The polymerization step occurred at 250 °C under reduced pressure for 2 h. The crude product was dissolved in 1,1,1,3,3,3-hexafluoroisopropanol then precipitated in cold methanol. The powder was vacuum dried at 60 °C for several days to afford the product (1.7 g, 88%). ¹H NMR (400 MHz, CF₃COOD:CDCl₃ 1:3, ppm): *δ* 7.52 (d, 2H, *J =* 3.5 Hz), 7.40 (d, 2H, *J =* 3.5 Hz), 4.76 (s, 4H).

*Resin curing experiment:* The diepoxy compound **4** was mixed with MHHPA in 1:1 molar ratio. A curing catalyst (0.5 wt%) dissolved in dichloromethane was then added. The mixture was homogenized by heating to 70 °C, followed by the application of vacuum (5 mbar) to remove the dichloromethane. The resin was transferred into a silicone mold and cured under reduced pressure in a vacuum oven (at 100, 120, and 150 °C, held 2 h at each temperature) to yield a dark solid.

*Dilute solution viscometry:* Intrinsic viscosities were determined from flow times of pure solvent (phenol:1,1,2,2-tetrachloroethane 60:40, w/w) (*t*₀) and 0.5 g/dL polyester solutions (*t*) in a micro-Ubbelohde viscometer submerged in 30.0 °C water bath. The average of three flow times were used to calculate *t*₀ and *t*. Billmeyer equation was used to calculate the intrinsic viscosity according to ASTM D 4603 technique. (Farah et al., 2015)

*Differential scanning calorimetry* (DSC): Differential scanning calorimeter (Mettler Toledo DSC 821e) was used to investigate thermal behavior of the samples in the range of -10-250 °C under N₂ gas flow (50 cm³/min) with heating and cooling rates of 10 °C/min. Samples (ca. 2-3 mg) were weighed into a 40 *µ*L Al pans, which were then sealed with pierced lids.

*Film processing:* Fontijne hydraulic press (LabEcon 300) was used to prepare melt-pressed polymer films. The polyester (1-2 g) was melted between the pre-heated aluminum plates covered with PTFE-coated glass fiber sheets. The thickness was controlled using layers of the same sheet around the sample. The polyesters were melted at 20-30 °C above the expected melting temperatures for 5 minutes, and then pressed for 1 minute at 30 kN. Water-cooling circuit of the press was used to cool the sample.

*Tensile testing:* Rectangle-shaped specimens with 5 mm width and thicknesses between 100-200 *µ*m were stored for 48 h under stable conditions (23 °C, 50% RH) before the tests. Tensile testing (Instron 5544, USA) was performed under the same conditions using a gage length of 30 mm and crosshead speed of 5 mm/min.

*Dynamic Mechanical Analysis* (DMA): The dynamic mechanical properties of polyesters rectangular melt-pressed film pieces were evaluated using a DMA Q800 (TA Instruments, USA). All runs were performed in a "multi-frequency, strain" mode at 1 Hz, 0.08% strain, from 30 to 150 °C at a heating rate of 3 °C/min.

*Gas permeability analysis*: The oxygen transmission rate (OTR) of the melt-pressed films was measured using a MOCON OxTran 2/20 (23 °C and 0% (relative humidity) with specimen exposure area = 5 cm². OTR values were measured from duplicate samples.

*UV-vis:* Shimadzu UV-1800 spectrophotometer was used to acquire transmission spectra of films within 200-800 nm.

### Results and Discussion

Dimethyl 5,5'-sulfanediyldi(furan-2-carboxylate) **3** was synthesized according to Figure 1 with three steps. Dialdehyde **1** was received from 5-bromofurfural using nucleophilic substitution reaction in good yield. A selective oxidation of the aldehyde functionalities of compound **1** in the presence of H₂O₂/Et₃N gave dicarboxylic acid **2** in high 95% yield. A following esterification of the diacid **2** afforded the dimethyl ester monomer **3** in high 97% yield. Diester **3** was used to prepare several high molecular weight poly(alkylene sulfanediylbisfuranoate) polyesters **8a-c** (Figure 1, Table 1). The intrinsic viscosities of the polyesters (0.90-0.83 dL/g, Table 1) were in the range for high molecular weight polyesters.

Other diester monomers, dimethyl 5,5'-sulfonyldi(furan-2-carboxylate) **(6)** and dimethyl 5,5'-sulfinyldi(furan-2-carboxylate) **(7)** were received in the following ways. In the presence of H₂O₂/CH₃COOH, the sulfide unit of diacid **2** oxidized affording compound **5** with the desired sulfone unit in good yield. The following esterification of **5** gave the monomer **6** in high 93% yield. Whereas, oxidation of the sulfide unit of **3** with *m*-CPBA gave the sulfoxide unit bearing monomer **7.** In order to demonstrate the utilization of the novel monomers, compound **6** was polymerized with ethylene glycol to receive polyester **9** with intrinsic viscosity of 0.18 dL/g (Table 1).

**Table 1. Reaction parameters for polyesters 8a-c and 9**

| Polyester | Diol equiv | Titanium Wt.% (ppm) | Esterification Time (h) | Polymerization | | Yield (%) | IV (dL/g) |
|---|---|---|---|---|---|---|---|
| | | | | Time (h) | Temp (°C) | | |
| 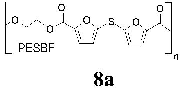 | 5 | 400 | 4 | 5 | 250 | 94 | 0.90 |
| 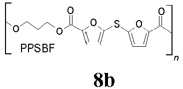 | 3 | 50 | 3 | 5 | 250 | 91 | 0.86 |
| 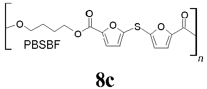 | 3 | 50 | 2 | 3 | 230 | 97 | 0.83 |
| 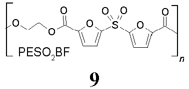 | 2 | 50 | 2 | 2 | 250 | 90 | 0.18 |

¹H and ¹³C NMR measurements confirmed the chemical structures. ¹H NMR spectra show two sets of duplets in the range of 6.5-7.5 ppm (*J =* 3.5 Hz). These are assigned to the furan ring, while the methylene protons are found at 1.6-4.7 ppm. ¹³C NMR spectra show the furan ring carbons (114-140 ppm) as well as the carbonyl carbon (156.7 ppm), with the alkyl chain carbons at 20-60 ppm.

The thermal properties of the polyesters are summarized in Table 2. Poly(alkylene sulfanediylbisfuranoate) polyesters showed lower glass transition temperature (*T*_{g}) values compared with those corresponding to poly(alkylene furanote) and poly(alkylene bifuranote). PESBF **(8a)** showed lower *T*_{g} (65 °C) than both poly(ethylene furanoate) (87 °C, Hong et al., 2016) and poly(ethylene bifuranoate) (107 °C, Kainulainen et al., 2018). Also, PBSBF **(8c)** with the lowest *T*_{g} value (37 °C) showed the same trend compared to poly(butylene furanoate) (39 °C) and poly(butylene bifuranoate) (62 °C) (Kainulainen et al., 2020). In contrast to sulfide polyesters, PESO₂BF **(9)** showed high glass transition temperature at 91 °C and melting temperature at 240 °C as sulfone groups give more strength to the polymer chains and restrict their free motion.

**Table 2. Thermal properties of polyesters 8a-c and 9**

| Sample | *T*_{g} (°C) | *T*ₘ (°C) | | *T*_{cc} (°C) |
|---|---|---|---|---|
| | | 1^{st} heating | 2^{nd} heating | |
| PESBF **(8a)** | 65 | 164 | nd | nd |
| PPSBF **(8b)** | 47 | 216 | 216 | 130 |
| PBSBF **(8c)** | 37 | 104, 134 | nd | nd |
| PESO₂BF **(9)** | 91 | 240 | nd | nd |

| | | | | |
|---|---|---|---|---|
| *T*_{g}: Glass transition temperature from 2^{nd} cooling cycle. *T*ₘ: Melting temperature from 1^{st} heating cycle. *T*_{cc}: Peak of cold crystallization from 2^{nd} heating cycle. nd: not determined. | | | | |

The viscoelastic properties of polyesters were analyzed by DMA. Notably, the three parameters storage modulus *E*', loss modulus *E*'' and tan *δ* for all samples showed the same general variation. It was found that both *E*'' and tan *δ* peak temperature values tended to decrease with increasing methylene unit count of the polyesters. The *T*_{g} values evaluated from the peak of the *E*'' curve were in line with those determined by DSC.

Polyesters **8a-c** were processed into free-standing films (thickness of 100-200 *µ*m) via melt-pressing. Tensile properties measured from the films (Table 3) fall within the same range as PBF, PEBF, and PBBF (Kainulainen et al., 2018 and 2020).

**Table 3. Measured tensile properties of polyesters 8a-c**

| Sample*^{a}* | *E*ₜ (MPa) | σₘ (MPa) | *ε*_{b} (%) |
|---|---|---|---|
| PESBF **(8a)** | 2300±200 | 30±8 | 1.3±0.3 |
| PPSBF **(8b)** | 2177± 100 | 42± 3 | 2.5±0.5 |
| PBSBF **(8c)** | 1410±30 | 33±6 | 12±6 |

| | | | |
|---|---|---|---|
| *^{a}*At least five amorphous specimens were evaluated for each polyester composition. *E*ₜ = Tensile modulus. σₘ = maximum tensile stress. *ε*_{b} = elongation at break. | | | |

Oxygen permeability coefficients were measured from films for both the polyesters **8a-c** and the reference material (PET) for determination of barrier improvement factors (BIFs, Table 4). Polyesters **8a-c** showed very high BIFs, which are in the same range as the previously reported values for PEF (Wang et al. 2017). They are higher compared to, e.g., the previously reported values for PBF, PEBF, and PBBF (Kainulainen et al., 2018 and 2020). Therefore, the novel sulfur-containing furan polyesters are excellent barrier materials.

**Table 4. O₂ barrier properties measured for novel polyesters 8a-c**

| Sample | Permeability coefficient (mL *µ*m m⁻² atm⁻¹ d⁻¹) | BIF vs. PET | Conditions |
|---|---|---|---|
| PET | 5760 | 1 | 23 °C at 0% RH |
| PESBF **(8a)** | 979 | 5.88 | |
| PPSBF **(8b)** | 984 | 5.85 | |
| PBSBF **(8c)** | 997 | 5.70 | |

| | | | |
|---|---|---|---|
| BIF = Barrier improvement factor. RH = Relative humidity | | | |

Based on UV-vis transmittance measurements, polyesters **8a-c** showed intrinsic UV screening up to ca. 350 nm. PET and FDCA-based polyesters do not provide notable screening in the UV-A region (Kainulainen et al., 2018 and 2020).

Diglycidyl ester **4** was prepared from diacid **2** via conversion into acyl chloride with thionyl chloride in dichloromethane, followed by reaction with glycidol and triethylamine. Diglycidyl ester **4** had a melting point of 70 °C (DSC). ¹H NMR spectrum was consistent with the expected product. In one curing experiment, compound **4** was mixed with hexahydromethylphthalic anhydride (MHHPA) in 1:1 molar ratio, with 0.5 wt% imidazole catalyst. The resulting mixture was held under vacuum after transfer into mold and thermally cured at up to 150 °C. DSC experiments showed that the resulting fully cured resin had a *T*_{g} of ca 120 °C.

### Conclusions

Furfural can be used as a feedstock for a novel class of polymers, where the key aromatic monomer incorporates a sulfur bridge between two furan rings. In simple linear polyesters, the key monomer structure resulted in amorphous or semi-crystalline polyesters with glass transition temperature ranging from 37-91 °C. The tested polyesters had excellent gas barrier properties against oxygen and intrinsic UV block up to about 350 nm.

In anhydride hardened epoxy resins, glass transition temperature of ca. 120 °C was observed. This closely matches the glass transition temperatures attained with diepoxy compounds that are derived from non-renewable terephthalic acid.

### REFERENCES

Kainulainen, T. P., Sirviö, J. A., Sethi, J., Hukka, T. I., Heiskanen, J. P. UV-Blocking Synthetic Biopolymer from Biomass-Based Bifuran Diester and Ethylene Glycol., Macromolecules, 2018, 51, 1822-1829.
Kainulainen, T. P.; Hukka, T. I.; Özeren, H. D.; Sirviö, J. A.; Hedenqvist, M. S.; Heiskanen, J. P. Utilizing Furfural-Based Bifuran Diester as Monomer and Comonomer for High-Performance Bioplastics: Properties of Poly(Butylene Furanoate), Poly(Butylene Bifuranoate), and Their Copolyesters. Biomacromolecules 2020, 21, 743-752.
Hong, S.; Min, K.-D.; Nam, B.-U.; Park, O. O. High molecular weight bio furan-based copolyesters for food packaging applications: synthesis, characterization and solid-state polymerization. Green Chem. 2016, 18, 5142-5150.
Wang, J.; Liu, X.; Zhu, J.; Jiang, Y. Copolyesters Based on 2,5-Furandicarboxylic Acid (FDCA): Effect of 2,2,4,4-Tetramethyl-1,3-Cyclobutanediol Units on Their Properties. Polymers 2017, 9, 305-319.
Ahmed, A. M.; Kainulainen, T. P.; Heiskanen, J. P. Furfural-Based Modification of PET for UV-Blocking Copolymers with Decreased Oxygen Permeability. Ind. Eng. Chem. Res. 2021, 60, 7495-7504.
Farah, S.; Kunduru, K. R.; Basu, A.; Domb, A. J. Molecular Weight Determination of Polyethylene Terephthalate; Elsevier Inc., 2015.

## Claims

1. A sulfur-bridged difuran polyester comprising:
a dicarboxylate component,
a diol component,
wherein the dicarboxylate component and/or the diol component comprise(s) a sulfur-bridged difuran structure.

2. The polyester according to claim 1, wherein said dicarboxylate component is selected from the group of the following formulas: 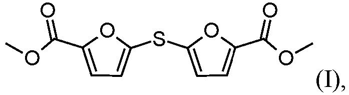 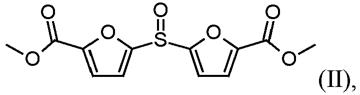 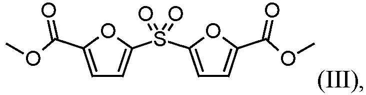 and combinations thereof.

3. The polyester according to claim 1, wherein said diol component is selected from the group of the following diols: 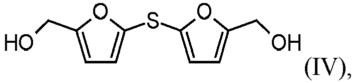 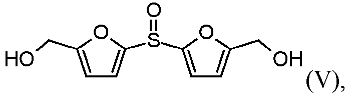 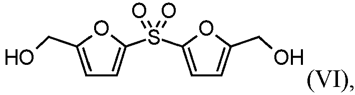 and combinations thereof.

4. The polyester according to claim 1, wherein said polyester is a homopolyester comprising repeating units of one of the structures according to formula 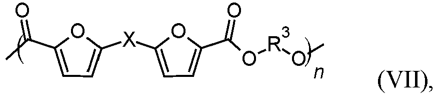
wherein X is independently selected from the group consisting of: -S-, -SO-, and -SO₂-; wherein R³ is independently selected from the group consisting of: -(CH₂)₂-, -(CH₂)₃-, - (CH₂)₄-, -(CH₂)₅-, -(CH₂)₆-, -(CH₂)₇-, -(CH₂)₈-, 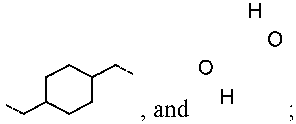 and
n is integer 1 or more.

5. The polyester according to claim 1, wherein said polyester is a copolyester and said diol component is an aliphatic, cycloaliphatic or aromatic C₂-C₈ diol, wherein said aliphatic or cycloaliphatic diol is preferably selected from the group consisting of: ethylene glycol, 1,3-propanediol, 1,4-butanediol, 1,4-cyclohexanedimethanol, and isosorbide.

6. The polyester according to any one of claims 1 or 5, wherein said polyester is a copolyester further comprising an aliphatic or cycloaliphatic C₃-C₈ dicarboxylic residue, an aromatic C₆-C₁₂ dicarboxylic residue or an aromatic C₆-C₈ dicarboxylic monomer residue as a repeating unit, wherein said aromatic C₆-C₈ dicarboxylic monomer residue is preferably dimethyl terephthalate (DMT).

7. The polyester according to claim 6, wherein said polyester is a copolyester comprising repeating units of at least one of the structures according to formula 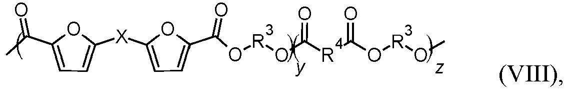
wherein X is selected from the group consisting of -S-, -SO-, and -SO₂-;
wherein each R³ is independently selected from the group consisting of -(CH₂)₂-, - (CH₂)₃-, -(CH₂)₄-, -(CH₂)₅-, -(CH₂)₆-, -(CH₂)₇-, -(CH₂)₈-, 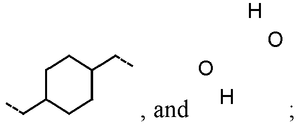 and
wherein R⁴ is selected from the group consisting of -(CH₂)₂-, -(CH₂)₃-, -(CH₂)₄-, - (CH₂)₅-, -(CH₂)₆-, and the following cyclic ring structures 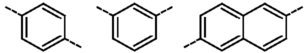 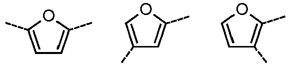 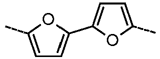 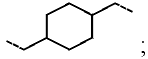 and
y and z are independently integer 1 or more.

8. The polyester according to claim 7, wherein the ratio of y:z is between 200:1 and 1:200, preferably 90:10, 75:25, 50:50, 25:75 or 10:90.

9. A package or packaging material comprising a sulfur-bridged difuran polyester according to any one of claims 1-8, wherein said package is preferably a food package, a beverage container, a cosmetics package, a detergent or a washing agent container, or a medicinal substance container.

10. A film, coating, or a yarn fiber comprising or consisting of a sulfur-bridged difuran polyester according to any one of claims 1-8.

11. Method for preparing a sulfur-bridged difuran, the method comprising the steps of:
a) reacting halogenated furfurals according to Formula IX 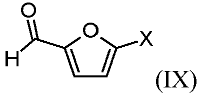
wherein X is independently Cl, Br or I;
with a sulfur donor in order to produce a sulfur-bridged difuran having the Formula X 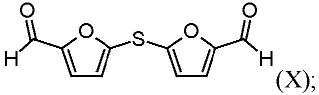
b) selectively oxygenating the sulfur-bridged difuran obtained from step a);
c) subjecting the oxygenated sulfur-bridged difuran thus obtained to esterification reaction in order to produce at least one of the following compounds: 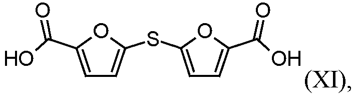 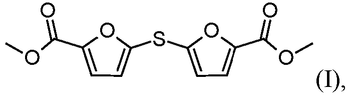 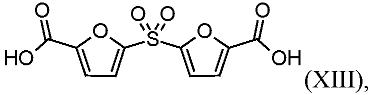 and

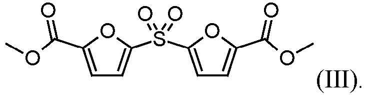

12. The method according to claim 11, the method comprising the steps of:
a) reacting halogenated furfurals according to Formula IX 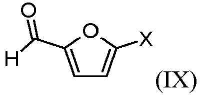
wherein X is independently Cl, Br or I;
with a sulfur donor in order to produce a sulfur-bridged difuran having the Formula X 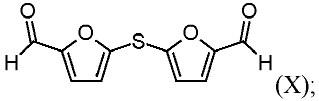
b) selectively oxygenating the aldehyde groups of the sulfur-bridged difuran obtained from step a) in order to produce a sulfur-bridged difuran having the Formula XI; 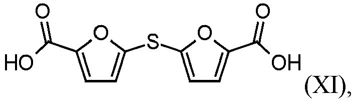
c) subjecting the oxygenated sulfur-bridged difuran thus obtained from step b) to esterification reaction in order to produce a sulfur-bridged difuran having the Formula I:

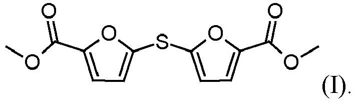

13. The method according to claim 11, the method comprising the steps of:
a) reacting halogenated furfurals according to Formula IX 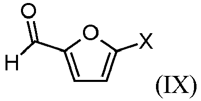
wherein X is independently Cl, Br or I;
with a sulfur donor in order to produce a sulfur-bridged difuran having the Formula X 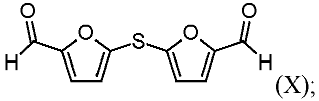
b) selectively oxygenating the aldehyde groups of the sulfur-bridged difuran obtained from step a) in order to produce a sulfur-bridged difuran having the Formula XI; 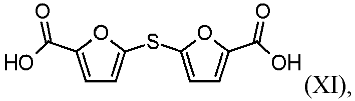
c) selectively oxygenating the sulfur atom of the sulfur-bridged difuran obtained from step b) in order to produce a sulfur-bridged difuran having the Formula XIII; 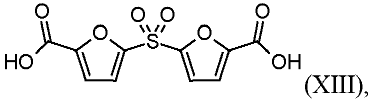
d) subjecting the oxygenated sulfur-bridged difuran thus obtained from step c) to esterification reaction in order to produce a sulfur-bridged difuran having the Formula III:

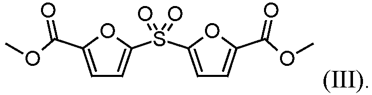

14. Method for preparing a sulfoxide-bridged difuran, the method comprising the steps of:
a) reacting halogenated furfurals according to Formula IX 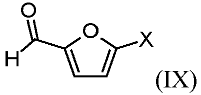
wherein X is independently Cl, Br or I;
with a sulfur donor in order to produce a sulfur-bridged difuran having the Formula X 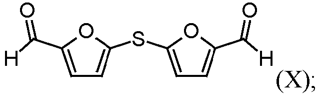
b) selectively oxygenating the aldehyde groups of the sulfur-bridged difuran obtained from step a);
c) esterifying the sulfur-bridged difuran diacid obtained from step b);
d) subjecting the esterified sulfur-bridged difuran thus obtained to oxygenation reaction in order to produce one of the following compounds: 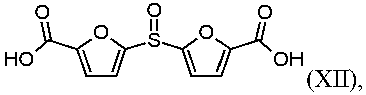 and

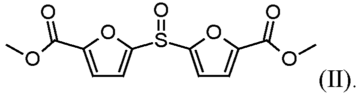

15. The method according to any one of claims 11-14, wherein said sulfur donor is sodium sulfide (Na₂S).

16. The method according to any one of claims 11-14, further comprising a step of performing a polycondensation process to any of the compounds according to Formulas I-III and XI-XIII or combinations thereof in order to produce a polyester.

17. The method according to claim any one of claims 11-14, wherein the compound of 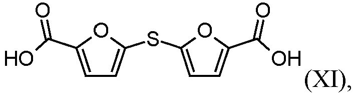 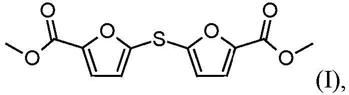 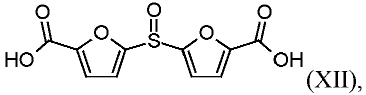 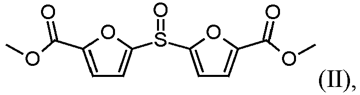 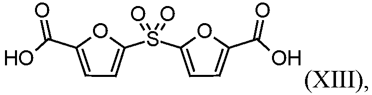 or 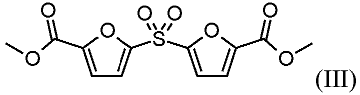 is further reacted with any of the following compounds or combinations thereof: 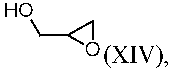 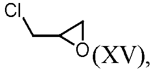 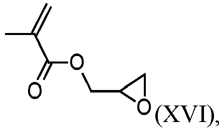 and

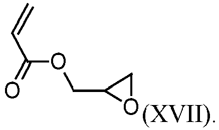

18. The method according to any one of claims 11-14, wherein the compound of 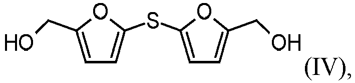 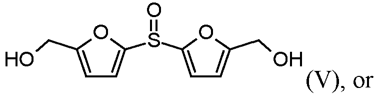 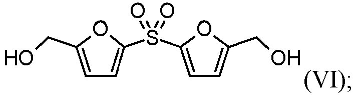 is reacted with any of the following compounds or combinations thereof: 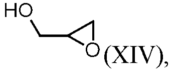 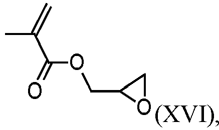 and

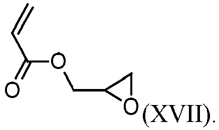

19. Method for preparing a sulfur-bridged difuran diol:
reacting the sulfur-bridged difuran aldehyde or ester according to Formula 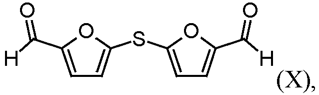 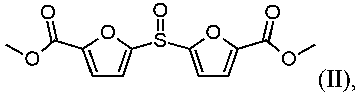 or 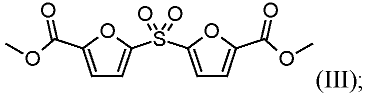
with sodium borohydride (NaBH₄) or lithium aluminum hydride (LiAlH₄) in order to produce one of the following compounds: 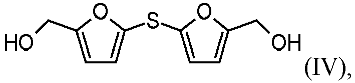 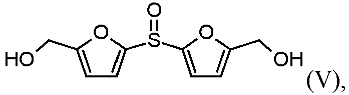 and

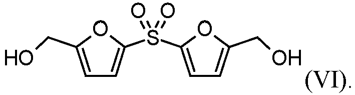

20. A sulfur-bridged difuran compound comprising or consisting of a structure of any of the following formulas: 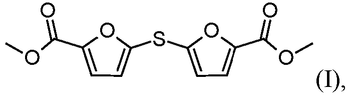 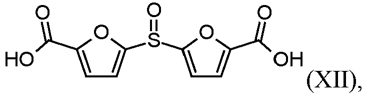 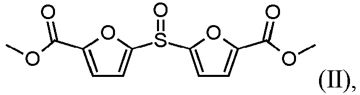 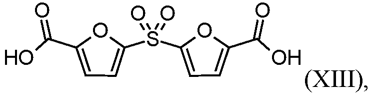 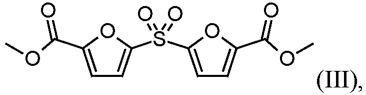 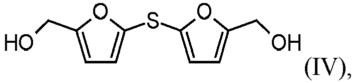 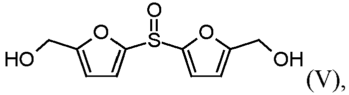 and

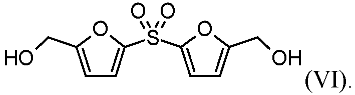

21. Use of the sulfur-bridged difuran compound according claim 20 in preparation of polyester with improved oxygen barrier and/or ultraviolet light (UV) blocking properties compared to poly(ethylene terephthalate) polymer.

22. A curable resin composition comprising at least one of the following sulfur-bridged difuran compounds: 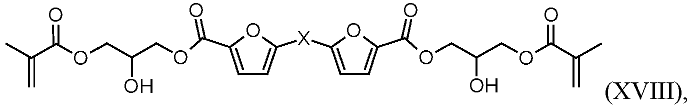 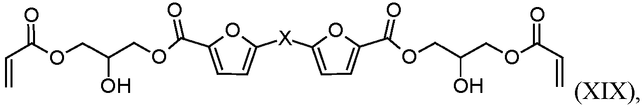 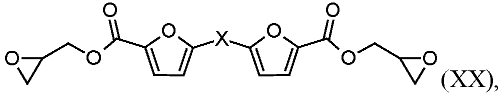 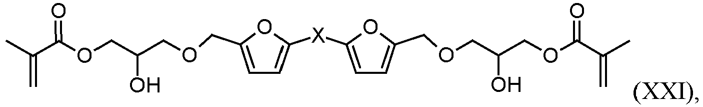 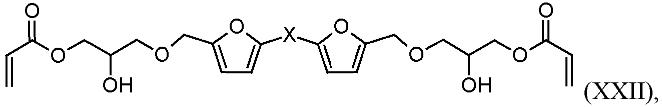 and 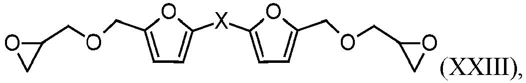 wherein X is selected from the group consisting of -S-, -SO-, and -SO₂-, wherein the composition is preferably cured by heating, or by UV radiation.

23. A composite, an adhesive, or a coating comprising at least one sulfur-bridged difuran compounds as defined in claim 22.

## Patentansprüche

1. Schwefelverbrückter Difuranpolyester, umfassend:
eine Dicarboxylatkomponente,
eine Diolkomponente,
wobei die Dicarboxylatkomponente und/oder die Diolkomponente eine schwefelverbrückte Difuranstruktur umfasst/umfassen.

2. Polyester nach Anspruch 1, wobei die Dicarboxylatkomponente aus der Gruppe der folgenden Formeln: 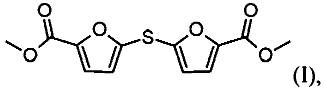 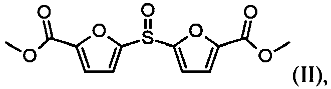 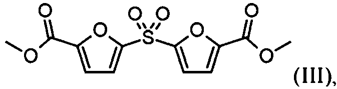 und Kombinationen davon ausgewählt ist.

3. Polyester nach Anspruch 1, wobei die Diolkomponente aus der Gruppe der folgenden Diole: 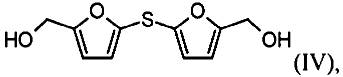 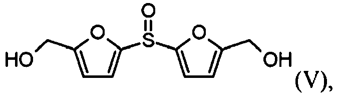 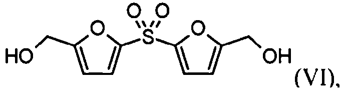 und Kombinationen davon ausgewählt ist.

4. Polyester nach Anspruch 1, wobei der Polyester ein Homopolyester ist, der Wiederholungseinheiten einer der Strukturen gemäß der Formel 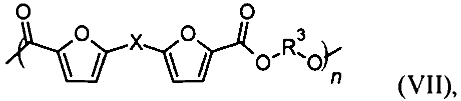
umfasst, wobei X unabhängig aus der Gruppe bestehend aus: -S-, -SO-, und -SO₂-ausgewählt ist;
wobei R³ unabhängig aus der Gruppe bestehend aus: -(CH₂)₂-, -(CH₂)₃-, - (CH₂)₄-, - (CH₂)₅-, -(CH₂)₆-, -(CH₂)₇-, -(CH₂)₈- 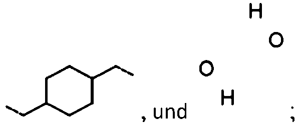 und
n Ganzzahl von 1 oder mehr ist.

5. Polyester nach Anspruch 1, wobei der Polyester ein Copolyester ist, und die Diolkomponente ein aliphatisches, cycloaliphatisches oder aromatisches C₂-C₈-Diol ist, wobei das aliphatische oder cycloaliphatische Diol bevorzugt aus der Gruppe bestehend aus: Ethylenglykol, 1,3-Propandiol, 1,4-Butandiol und 1,4-Cyclohexandimethanol und Isosorbid ausgewählt ist.

6. Polyester nach einem der Ansprüche 1 oder 5, wobei der Polyester ein Copolyester ist, der weiter einen aliphatischen oder cycloaliphatischen C₃-C₈-Dicarbonsäurerest, einen aromatischen C₆-C₁₂-Dicarbonsäurerest oder einen aromatischen C₆-C₈-Dicarbonsäuremonomerrest als Wiederholungseinheit umfasst, wobei der aromatische C₆-C₈-Dicarbonsäuremonomerrest bevorzugt Dimethylterephthalat (DMT) ist.

7. Polyester nach Anspruch 6, wobei der Polyester ein Copolyester ist, der Wiederholungseinheiten von mindestens einer der Strukturen gemäß der Formel 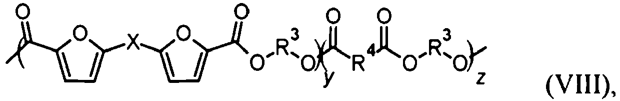
umfasst, wobei X aus der Gruppe bestehend aus: -S-, -SO-, und -SO₂- ausgewählt ist;
wobei jedes R³ unabhängig aus der Gruppe bestehend aus -(CH₂)₂-, -(CH₂)₃-, - (CH₂)₄-, -(CH₂)₅-, -(CH₂)₆-, -(CH₂)₇-, -(CH₂)₈-, 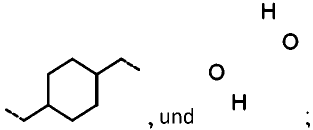 und
wobei R⁴ aus der Gruppe bestehend aus -(CH₂)₂-, -(CH₂)₃-, -(CH₂)₄-, -(CH₂)₅-, - (CH₂)₆-, und den folgenden zyklischen Ringstrukturen 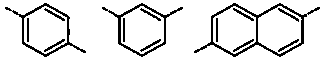 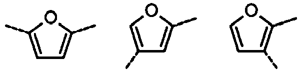 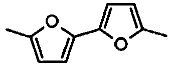 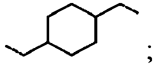 und
y und z unabhängig Ganzzahl größer oder gleich 1 sind.

8. Polyester nach Anspruch 7, wobei das Verhältnis von y:z zwischen 200:1 und 1:200, bevorzugt 90:10, 75:25, 50:50, 25:75 oder 10:90, ist.

9. Verpackung oder Verpackungsmaterial, umfassend einen schwefelverbrückten Difuranpolyester nach einem der Ansprüche 1-8, wobei die Verpackung bevorzugt eine Lebensmittelverpackung, einen Getränkebehälter, eine Kosmetikverpackung, einen Waschmittel- oder Reinigungsmittelbehälter oder ein Behälter für medizinische Substanzen ist.

10. Film, Beschichtung oder Garnfaser, die einen schwefelverbrückten Difuranpolyester nach einem der Ansprüche 1-8 umfasst oder daraus besteht.

11. Verfahren zur Vorbereitung eines schwefelverbrückten Difurans, wobei das Verfahren die folgenden Schritte umfasst:
a) Reagieren halogenierter Furfurale gemäß Formel IX 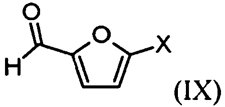
wobei X unabhängig Cl, Br oder I ist;
mit einem Schwefeldonor, um ein schwefelverbrücktes Difuran herzustellen, das die Formel X aufweist: 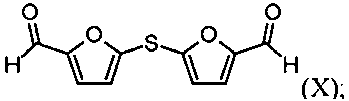
b) selektives Sauerstoffanreichern des aus Schritt a) erhaltenen schwefelverbrückten Difurans;
c) Unterziehen des so erhaltenen sauerstoffhaltigen, schwefelverbrückten Difurans einer Veresterungsreaktion, um mindestens eine der folgenden Verbindungen herzustellen: 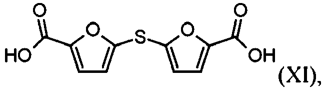 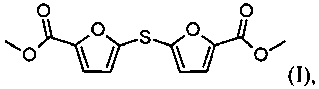 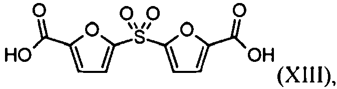 und

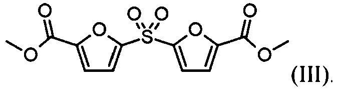

12. Verfahren nach Anspruch 11, wobei das Verfahren die folgenden Schritte umfasst:
a) Reagieren halogenierter Furfurale gemäß Formel IX 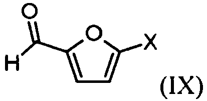
wobei X unabhängig Cl, Br oder I ist;
mit einem Schwefeldonor, um ein schwefelverbrücktes Difuran herzustellen, das die Formel X aufweist: 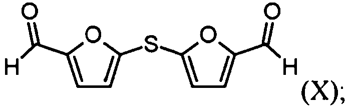
b) selektives Sauerstoffanreichern der Aldehydgruppen des aus Schritt a) erhaltenen schwefelverbrückten Difurans, um ein schwefelverbrücktes Difuran herzustellen, das die Formel XI aufweist; 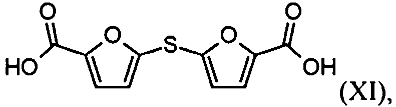
c) Unterziehen des so aus Schritt b) erhaltenen sauerstoffhaltigen, schwefelverbrückten Difurans einer Veresterungsreaktion, um ein schwefelverbrücktes Difuran herzustellen, das die Formel I aufweist:

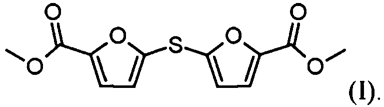

13. Verfahren nach Anspruch 11, wobei das Verfahren die folgenden Schritte umfasst:
a) Reagieren halogenierter Furfurale gemäß Formel IX 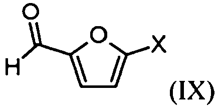
wobei X unabhängig Cl, Br oder I ist;
mit einem Schwefeldonor, um ein schwefelverbrücktes Difuran herzustellen, das die Formel X aufweist: 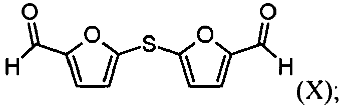
b) selektives Sauerstoffanreichern der Aldehydgruppen des aus Schritt a) erhaltenen schwefelverbrückten Difurans, um ein schwefelverbrücktes Difuran herzustellen, das die Formel XI aufweist; 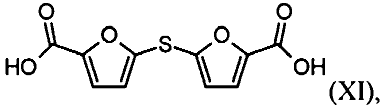
c) selektives Sauerstoffanreichern des Schwefelatoms des aus Schritt b) erhaltenen schwefelverbrückten Difurans, um ein schwefelverbrücktes Difuran herzustellen, das die Formel XIII aufweist; 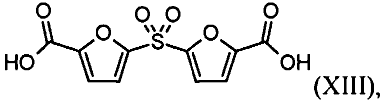
d) Unterziehen des so aus Schritt c) erhaltenen sauerstoffhaltigen, schwefelverbrückten Difurans einer Veresterungsreaktion, um ein schwefelverbrücktes Difuran herzustellen, das die Formel III aufweist:

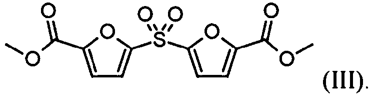

14. Verfahren zur Vorbereitung eines sulfoxidverbrückten Difurans, wobei das Verfahren die folgenden Schritte umfasst:
a) Reagieren halogenierter Furfurale gemäß Formel IX 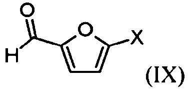
wobei X unabhängig Cl, Br oder I ist;
mit einem Schwefeldonor, um ein schwefelverbrücktes Difuran herzustellen, das die Formel X aufweist: 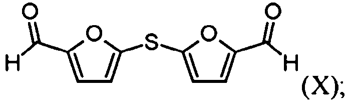
b) selektives Sauerstoffanreichern der Aldehydgruppen des aus Schritt a) erhaltenen schwefelverbrückten Difurans;
c) Verestern der aus Schritt b) erhaltenen schwefelverbrückten Difurandisäure;
d) Unterziehen des so erhaltenen veresterten schwefelverbrückten Difurans einer Sauerstoffanreicherungsreaktion, um eine der folgenden Verbindungen herzustellen: 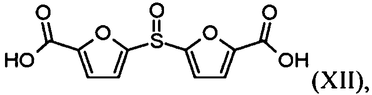 und

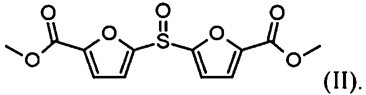

15. Verfahren nach einem der Ansprüche 11-14, wobei der Schwefeldonor Natriumsulfid (Na₂S) ist.

16. Verfahren nach einem der Ansprüche 11-14, das weiter einen Schritt zum Durchführen eines Polykondensationsprozesses mit einer der Verbindungen gemäß den Formeln I-III und XI-XIII oder Kombinationen davon umfasst, um einen Polyester herzustellen.

17. Verfahren nach einem der Ansprüche 11-14, wobei die Verbindung von 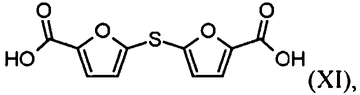 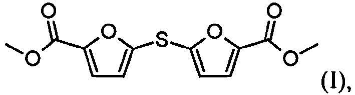 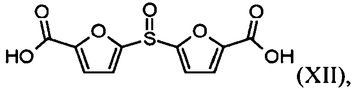 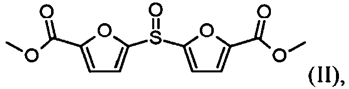 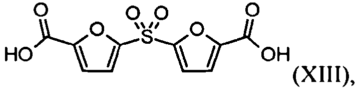 ode 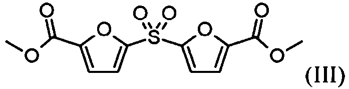 weiter mit einer der folgenden Verbindungen oder Kombinationen davon reagiert: 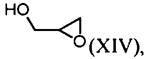 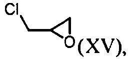 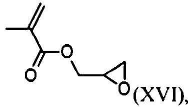 und

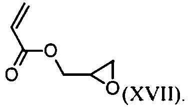

18. Verfahren nach einem der Ansprüche 11-14, wobei die Verbindung von 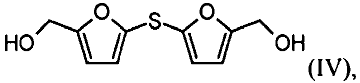 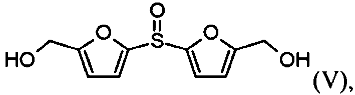 ode 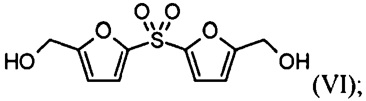 mit einer der folgenden Verbindungen oder Kombinationen davon reagiert: 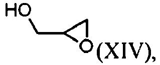 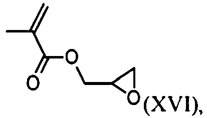 und

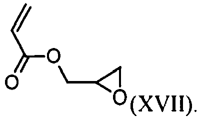

19. Verfahren zur Vorbereitung eines schwefelverbrückten Difurandiols:
Reagieren des schwefelverbrückten Difuranaldehyds oder -esters gemäß Formel 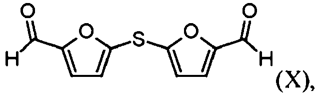 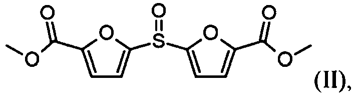 ode 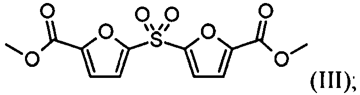
mit Natriumborhydrid (NaBH₄) oder Lithiumaluminiumhydrid (LiAlH₄), um eine der folgenden Verbindungen herzustellen: 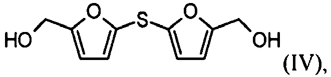 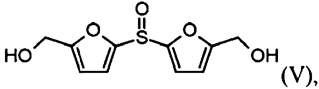 und

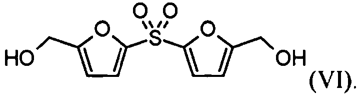

20. Schwefelverbrückte Difuranverbindung, die eine Struktur mit einer der folgenden Formeln umfasst oder daraus besteht: 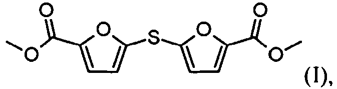 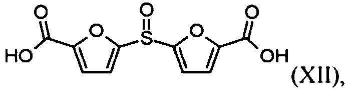 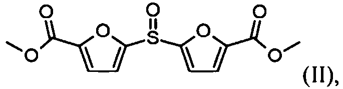 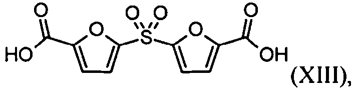 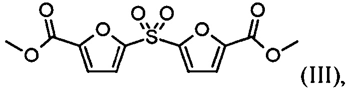 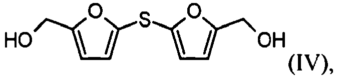 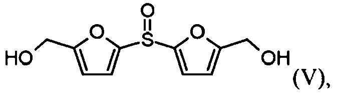 und

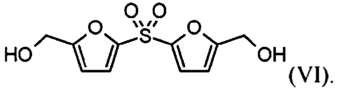

21. Verwendung der schwefelverbrückten Difuranverbindung nach Anspruch 20 zur Vorbereitung von Polyester mit verbesserter Sauerstoffbarriere und/oder UVblockierenden Eigenschaften im Vergleich zu Poly(ethylenterephthalat)-Polymer.

22. Härtbare Harzzusammensetzung, die mindestens eine der folgenden schwefelverbrückten Difuranverbindungen umfasst: 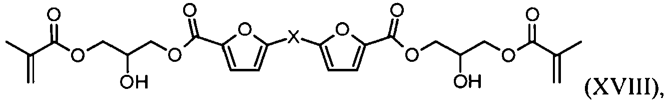 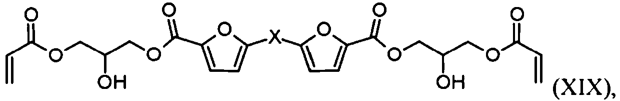 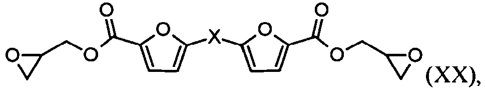 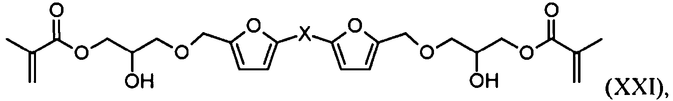 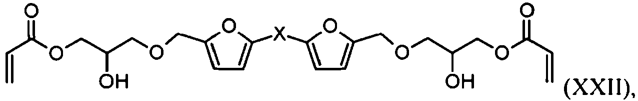 und 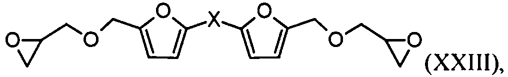 wobei X aus der Gruppe bestehend aus -S-, -SO-, und -SO₂- ausgewählt ist, wobei die Zusammensetzung bevorzugt durch Erhitzen oder durch UV-Strahlung ausgehärtet wird.

23. Verbundwerkstoff, Klebstoff oder Beschichtung, umfassend mindestens eine der schwefelverbrückten Difuranverbindungen nach Anspruch 22.

## Revendications

1. Polyester de difurane ponté au soufre comprenant :
un composant dicarboxylate,
un composant diol,
dans lequel le composant dicarboxylate et/ou le composant diol comprend(comprennent) une structure de difurane pontée au soufre.

2. Polyester selon la revendication 1, dans lequel ledit composant dicarboxylate est choisi dans le groupe de composants des formules suivantes : 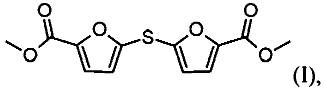 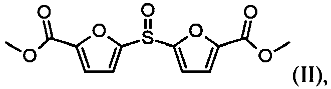 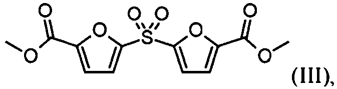 et des combinaisons de ceux-ci.

3. Polyester selon la revendication 1, dans lequel ledit composant diol est choisi dans le groupe des diols suivants : 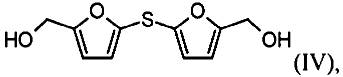 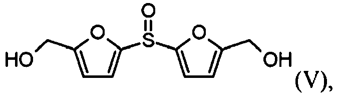 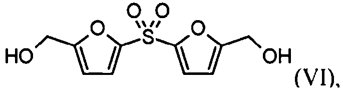 et des combinaisons de ceux-ci.

4. Polyester selon la revendication 1, dans lequel ledit polyester est un homopolyester comprenant des unités répétitives d'une des structures selon la formule 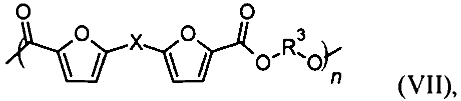
dans laquelle X est choisi indépendamment dans le groupe consistant en : -S-, -SO-, et -SO₂- ;
dans laquelle R³ est choisi indépendamment dans le groupe consistant en : -(CH₂)₂-, -(CH₂)₃-, - (CH₂)₄-, -(CH₂)₅-, -(CH₂)₆-, -(CH₂)₇-, -(CH₂)₈-, 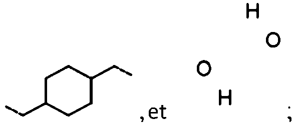 et
n est un entier supérieur ou égal à 1.

5. Polyester selon la revendication 1, dans lequel ledit polyester est un copolyester et ledit composant diol est un diol aliphatique, cycloaliphatique ou aromatique en C₂-C₈, dans lequel ledit diol aliphatique ou cycloaliphatique est de préférence choisi dans le groupe consistant en: éthylène glycol, 1,3-propanediol, 1,4-butanediol, 1,4-cyclohexanediméthanol et isosorbide.

6. Polyester selon l'une quelconque des revendications 1 et 5, dans lequel ledit polyester est un copolyester comprenant en outre un résidu dicarboxylique en C₃-C₈ aliphatique ou cycloaliphatique, un résidu dicarboxylique en C₆-C₁₂ aromatique ou un résidu monomère dicarboxylique en C₆-C₈ aromatique comme unité répétitive, dans lequel ledit résidu monomère dicarboxylique en C₆-C₈ aromatique est de préférence du téréphtalate de diméthyle (DMT).

7. Polyester selon la revendication 6, dans lequel ledit polyester est un copolyester comprenant des unités répétitives d'au moins une des structures selon la formule 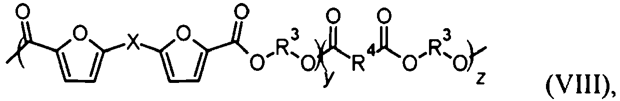
dans laquelle X est choisi dans le groupe consistant en -S-, -SO-, et -SO₂- ;
dans laquelle chaque R³ est choisi indépendamment dans le groupe consistant en - (CH₂)₂-, -(CH₂)₃-, -(CH₂)₄-, -(CH₂)₅-, -(CH₂)₆-, -(CH₂)₇-, -(CH₂)₈-, 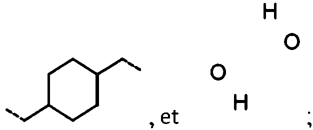 et
dans laquelle R⁴ est choisi dans le groupe consistant en -(CH₂)₂-, -(CH₂)₃-, -(CH₂)₄-, -(CH₂)₅-, -(CH₂)₆-, et des structures de cycle cycliques suivantes 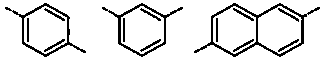 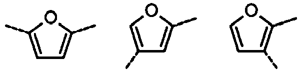 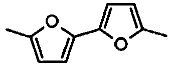 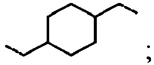 et
y et z sont indépendamment des entiers supérieurs ou égaux à 1.

8. Polyester selon la revendication 7, dans lequel le rapport de y:z est entre 200:1 et 1:200, de préférence de 90:10, 75:25, 50:50, 25:75 ou 10:90.

9. Emballage ou matériau d'emballage comprenant un polyester de difurane ponté au soufre selon l'une quelconque des revendications 1 à 8, dans lequel ledit emballage est de préférence un emballage alimentaire, un récipient de boisson, un emballage de cosmétique, un récipient de détergent ou d'agent de lavage, ou un récipient de substance médicinale.

10. Film, revêtement ou fibre de fil comprenant ou consistant en un polyester de difurane ponté au soufre selon l'une quelconque des revendications 1-8.

11. Procédé de préparation d'un difurane ponté au soufre, le procédé comprenant les étapes suivantes :
a) la réaction de furfurals halogénés selon la formule IX 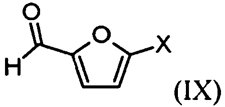
dans laquelle X est indépendamment Cl, Br ou I ;
avec un donneur de soufre afin de produire un difurane ponté au soufre présentant la formule X 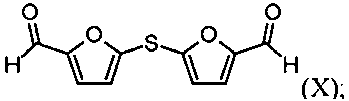
b) l'oxygénation sélective du difurane ponté au soufre obtenu à l'étape a) ;
c) la soumission du difurane oxygéné ponté au soufre ainsi obtenu à une réaction d'estérification afin de produire au moins l'un des composés suivants : 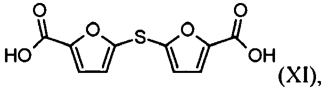 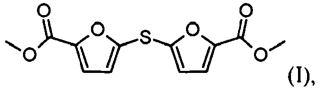 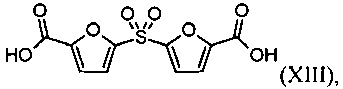 et

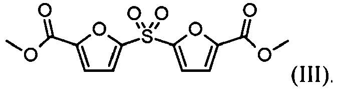

12. Procédé selon la revendication 11, le procédé comprenant les étapes suivantes :
a) la réaction de furfurals halogénés selon la formule IX 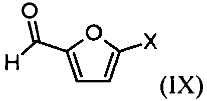
dans laquelle X est indépendamment CI, Br ou I ;
avec un donneur de soufre afin de produire un difurane ponté au soufre présentant la formule X 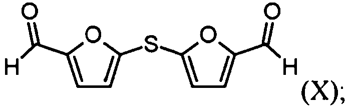
b) l'oxygénation sélective des groupes aldéhydes du difurane ponté au soufre obtenu à l'étape a) afin de produire un difurane ponté au soufre présentant la formule XI ; 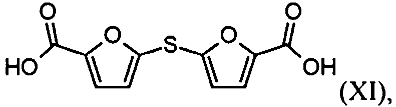
c) la soumission du difurane ponté au soufre oxygéné ainsi obtenu à l'étape b) à une réaction d'estérification afin de produire un difurane ponté au soufre présentant la formule I :

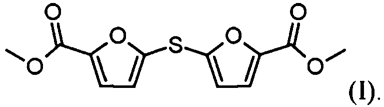

13. Procédé selon la revendication 11, le procédé comprenant les étapes suivantes :
a) la réaction de furfurals halogénés selon la formule IX 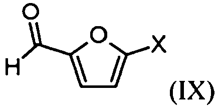
dans laquelle X est indépendamment Cl, Br ou I ;
avec un donneur de soufre afin de produire un difurane ponté au soufre présentant la formule X 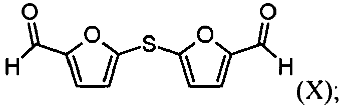
b) l'oxygénation sélective des groupes aldéhydes du difurane ponté au soufre obtenu à l'étape a) afin de produire un difurane ponté au soufre présentant la formule XI ; 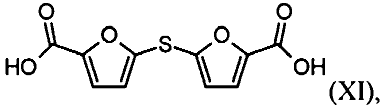
c) l'oxygénation sélective de l'atome de soufre du difurane ponté au soufre obtenu à l'étape b) afin de produire un difurane ponté au soufre présentant la formule XIII ; 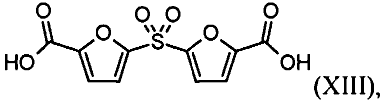
d) la soumission du difurane ponté au soufre oxygéné ainsi obtenu à l'étape c) à une réaction d'estérification afin de produire un difurane ponté au soufre présentant la formule III :

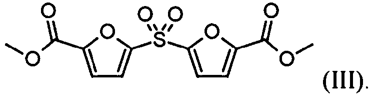

14. Procédé de préparation d'un difurane ponté au sulfoxyde, le procédé comprenant les étapes suivantes :
a) la réaction de furfurals halogénés selon la formule IX 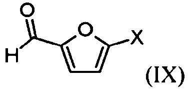
dans laquelle X est indépendamment CI, Br ou I ;
avec un donneur de soufre afin de produire un difurane ponté au soufre présentant la formule X 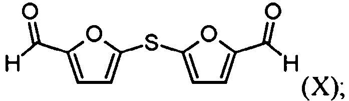
b) l'oxygénation sélective des groupes aldéhydes du difurane ponté au soufre obtenu à l'étape a) ;
c) l'estérification du diacide de difurane ponté au soufre obtenu à l'étape b) ;
d) la soumission du difurane ponté au soufre estérifié ainsi obtenu à une réaction d'oxygénation afin de produire l'un des composés suivants : 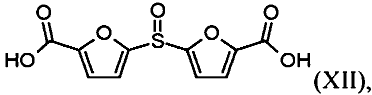 et

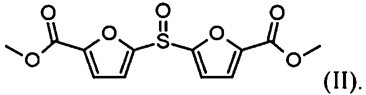

15. Procédé selon l'une quelconque des revendications 11-14, dans lequel ledit donneur de soufre est du sulfure de sodium (Na₂S).

16. Procédé selon l'une quelconque des revendications 11-14, comprenant en outre une étape de réalisation d'un processus de polycondensation sur l'un quelconque des composés selon les formules I-III et XI-XIII ou des combinaisons de ceux-ci afin de produire un polyester.

17. Procédé selon l'une quelconque des revendications 11-14, dans lequel le composé de 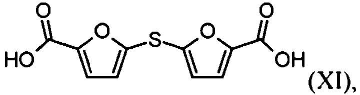 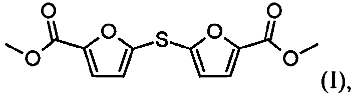 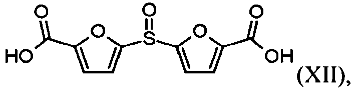 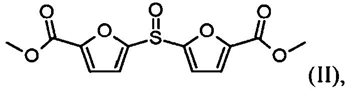 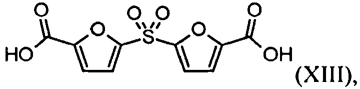 ou 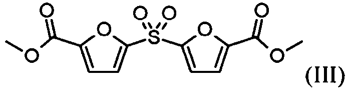 est en outre mis à réagir avec l'un des composés suivants ou avec une combinaison de ceux-ci : 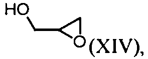 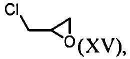 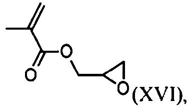 et

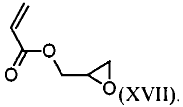

18. Procédé selon l'une quelconque des revendications 11-14, dans lequel le composé de 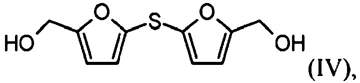 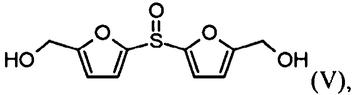 ou 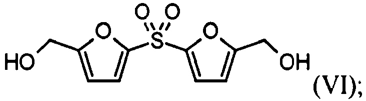 est mis à réagir avec l'un quelconque des composés suivants ou des combinaisons de ceux-ci : 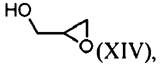 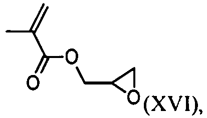 et

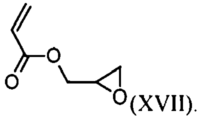

19. Procédé de préparation d'un difurane diol ponté au soufre :
la réaction de l'aldéhyde ou l'ester de difurane ponté au soufre selon la formule 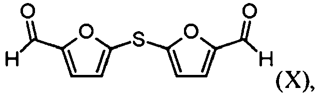 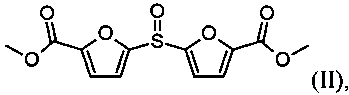 ou 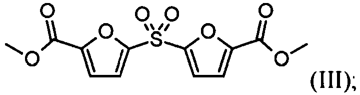
avec du borohydrure de sodium (NaBH₄) ou de l'hydrure de lithium et d'aluminium (LiAlH₄) afin de produire l'un des composés suivants :

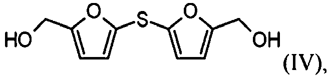

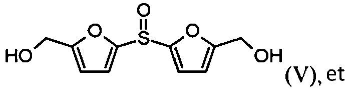

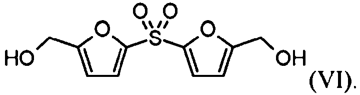

20. Composé de difurane ponté au soufre comprenant ou consistant en une structure selon l'une quelconque des formules suivantes : 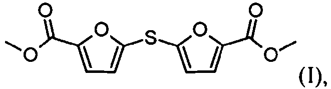 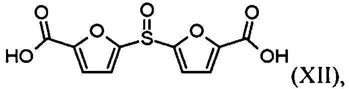 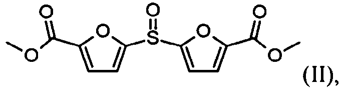 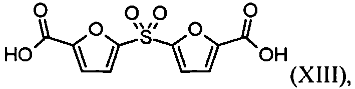 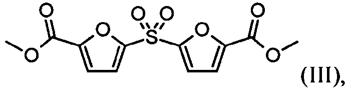 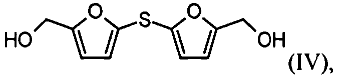 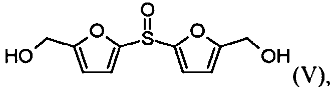 et

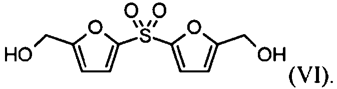

21. Utilisation du composé de difurane ponté au soufre selon la revendication 20 dans la préparation de polyester avec des propriétés améliorées de barrière à l'oxygène et/ou de blocage de la lumière ultraviolette (UV) par rapport au polymère poly(éthylène téréphtalate).

22. Composition de résine durcissable comprenant au moins l'un des composés de difurane ponté au soufre suivants : 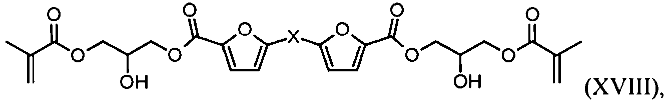 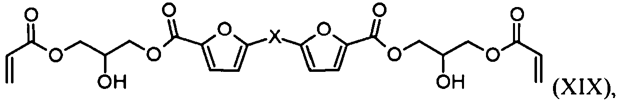 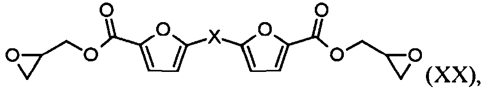 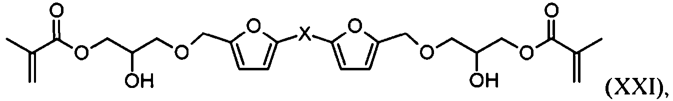 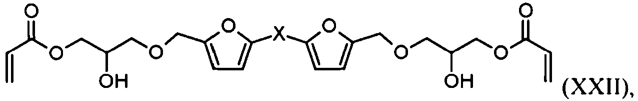 et 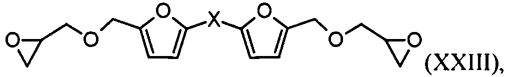 dans laquelle X est choisi dans le groupe consistant en -S-, -SO- et -SO₂-, dans laquelle la composition est de préférence durcie par chauffage ou par rayonnement UV.

23. Composite, adhésif ou revêtement comprenant au moins un composé de difurane ponté au soufre tel que défini dans la revendication 22.
